(19) Europäisches Patentamt
European Patent Office
Office européen des brevets

(11) EP 3 729 073 B1

(12) FASCICULE DE BREVET EUROPEEN

(45) Date de publication et mention
de la délivrance du brevet:
**08.06.2022 Bulletin 2022/23**

(21) Numéro de dépôt: **18842796.7**

(22) Date de dépôt: **21.12.2018**

(51) Classification Internationale des Brevets (IPC):
*G01N 33/00* (2006.01)    *A01M 31/00* (2006.01)

(52) Classification Coopérative des Brevets (CPC):
**G01N 33/0047; A01M 31/002**

(86) Numéro de dépôt international:
**PCT/FR2018/053509**

(87) Numéro de publication internationale:
**WO 2019/122781 (27.06.2019 Gazette 2019/26)**

(54) **PROCÉDÉ DE DÉTECTION D'UNE INFESTATION D'INSECTES**

VERFAHREN ZUM NACHWEIS EINES INSEKTENBEFALLS

METHOD OF DETECTING AN INSECT INFESTATION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.12.2017 FR 1762893**

(43) Date de publication de la demande:
**28.10.2020 Bulletin 2020/44**

(73) Titulaire: **Centre Scientifique Et Technique Du Batiment (CSTB)
77420 Champs sur Marne (FR)**

(72) Inventeurs:
• **LACAZE, Isabelle**
  **77500 Chelles (FR)**
• **MOULARAT, Stéphane**
  **77200 Torcy (FR)**
• **ROBINE, Enric**
  **77600 Conches-sur-Gondoire (FR)**

(74) Mandataire: **Plasseraud IP
66, rue de la Chaussée d'Antin
75440 Paris Cedex 09 (FR)**

(56) Documents cités:
**FR-A1- 2 913 501**

• **SAI XU ET AL: "Recognition of the Duration and Prediction of Insect Prevalence of Stored Rough Rice Infested by the Red Flour Beetle (Tribolium castaneum Herbst) Using an Electronic Nose", SENSORS, vol. 17, no. 4, 14 avril 2017 (2017-04-14) , page 688, XP055507460, DOI: 10.3390/s17040688**
• **BRITTANY D. LAMPSON ET AL: "Development of a Portable Electronic Nose for Detection of Cotton Damaged by Nezara viridula (Hemiptera: Pentatomidae)", JOURNAL OF INSECTS, vol. 2014, 11 novembre 2014 (2014-11-11), pages 1-8, XP055507458, ISSN: 2356-7465, DOI: 10.1155/2014/297219**
• **J WU ET AL: "Feasibility of the application of electronic nose technology to detect insect infestation in wheat", CANADIAN BIOSYSTEMS ENGINEERING, vol. 55, no. 1, 31 décembre 2013 (2013-12-31), pages 3.1-3.9, XP055507457, CA ISSN: 1492-9058, DOI: 10.7451/CBE.2013.55.3.1**

**Description**

La présente invention concerne un procédé de détection d'une infestation d'insectes, y compris à l'état de larves dans des environnements intérieurs.

Par environnement intérieur on entend un espace clos ou confiné à l'intérieur d'un bâtiment qui est aéré de façon non continue (*via* les ouvrants...) ou de façon continue par une ventilation forcée. Des exemples d'environnements intérieurs peuvent être trouvés dans les habitations, les musées, les églises, les caves, les monuments historiques, les bâtiments administratifs, les écoles et les hôpitaux.

Les insectes sont des ravageurs redoutés dans de nombreux espaces clos. Ils s'attaquent notamment aux charpentes, meubles, boiseries, tissus, denrées ... et provoquent des dégradations irréversibles aboutissant à la perte de résistance mécanique des éléments du bâti, à la destruction d'objets patrimoniaux ou encore à la perte de céréales stockées.

Les techniques actuelles permettant de les détecter reposent essentiellement sur la détection de l'insecte adulte, après dégradation, et non à l'état de larve pourtant responsable des dégâts. Ainsi, les techniques classiques ne permettent pas de mettre en place une stratégie préventive permettant de traiter les environnements sinon de constater une dégradation effective.

Dans ce contexte, la détection précoce de ces insectes ravageurs constitue un objectif majeur pour prévenir la dégradation de l'habitat, des objets patrimoniaux ou des denrées alimentaires et pour limiter ainsi l'emploi de traitements curatifs et les coûts élevés associés de réhabilitation, de restauration, de prévention etc.

Un des buts de la présente invention est donc de pouvoir détecter de façon précoce la présence d'insectes ravageurs, même à l'état de larves. On entend par détection « précoce » une détection de la présence d'insectes ravageurs avant l'apparition de signes visibles. La détection précoce est de grande importance dans les sites patrimoniaux comme les monuments historiques, les églises ou les musées, où des dommages irrémédiables ont en général déjà été causés quand les premiers signes visibles d'une infestation apparaissent.

Dans la demande FR 2 913 501 intitulée « Procédé de détection d'une contamination fongique » la Société Demanderesse a déterminé un « indice chimique de contamination fongique », dénommé « ICF », qui permet de détecter une contamination fongique dans un environnement intérieur.

Cependant, la méthode de calcul de l'ICF décrit dans ce document FR 2 913 501 ne permet pas d'être suffisamment spécifique concernant la détection des insectes. Elle n'est par conséquent pas transposable en l'état et nécessite d'être améliorée Il est aussi connu d'employer un nez électronique pour la détection de la présence d'insectes en analysant une signature des Composés Organiques Volatils (COV) dans un échantillon, comme décrit, par exemple, dans SAI XU ET AL: "Récognition of the Duration and Prediction of Insect Prevalence of Stored Rough Rice Infested by the Red Flour Beetle (Tribolium castaneum Herbst) Using an Electronic Nose",SENSORS, vol. 17, no. 4, 14 avril 2017 (2017-04-14), page 688, XP055507460,DOI: 10.3390/s17040688.

La Société Demanderesse a donc continué ses recherches en engageant des travaux basés sur la détection des larves à partir des Composés Organiques Volatils (COV) issus de leur métabolisme et/ou de la dégradation des supports. Ces COV cibles se dispersent dans l'environnement sans être retenus par les supports. Plus particulièrement, la Société Demanderesse a maintenant trouvé que certains COV n'étaient présents dans l'air ambiant qu'en présence d'insectes, y compris à l'état de larves. Ces COV sont donc spécifiquement issus du métabolisme des insectes, y compris à l'état de larves. Elle a également trouvé que certains autres COV étaient présents dans l'air ambiant non seulement en présence des insectes, mais aussi en présence de certains matériaux ou encore d'autres contaminations biologiques.

Forte de cette découverte, la Société Demanderesse a pu mettre en évidence que ces COV spécifiques des insectes peuvent être divisés en deux groupes :

-   les COV qui sont émis indépendamment du support sur lequel se développent les insectes, et
-   les COV qui sont émis en fonction du support sur lequel les insectes se développent.

Parmi les COV qui sont émis indépendamment du support, on distingue les COV qui ne sont émis que par des insectes et les COV qui peuvent avoir d'autres origines biologiques que les insectes.

Elle a ainsi déterminé trois catégories distinctes de COV. La détermination et la détection de ces COV spécifiques des insectes, encore dénommés COV « cibles » prédéterminés, est à la base de l'invention.

En effet, la détection de ces COV cibles prédéterminés permet d'une part la détection d'une infestation de manière précoce et d'autre part la détection des infestations non visibles à l'œil nu.

La Société Demanderesse a plus particulièrement mis au point une valeur « Indice Infestation Insecte » représentée par « 3I » » (encore appelé indice 3I) et une valeur « Valeur limite » représentée par « VL », qui sont dépendantes chacune de la présence ou de l'absence des COV cibles prédéterminés. La détermination et la comparaison de ces valeurs entre elles permet de détecter de façon précoce la présence d'insectes, même à l'état de larves.

La Société Demanderesse a donc le mérite d'avoir, après des travaux de recherche longs et approfondis, mis au point un procédé de détection d'une infestation d'insectes, y compris à l'état de larves, dans des environnements intérieurs même en l'absence de signes d'infestations visibles.

La présente invention a plus particulièrement pour objet un procédé de détection de la présence d'insectes dans un environnement intérieur comprenant :

a/ le prélèvement d'un échantillon de composés organiques volatils (COV) dans l'environnement intérieur ;
b/ la séparation des COV prélevés ;
c/ la détection de la présence ou de l'absence de COV « cibles » prédéterminés, ces COV « cibles » prédéterminés appartenant à au moins l'une des trois catégories de COV suivantes :

- les COV qui sont émis indépendamment du support sur lequel les insectes se développent et qui ne sont émis que lors d'une infestation par des insectes, dénommés « COV de la catégorie 1 » ;
- les COV qui sont émis indépendamment du support sur lequel les insectes se développent mais qui peuvent également avoir d'autres origines biologiques que les insectes, dénommés « COV de la catégorie 2 » ;
- les COV qui sont émis en fonction du support sur lequel les insectes se développent et qui ne sont émis que lors d'une infestation par des insectes, dénommés « COV de la catégorie 3 » ;

d/ le calcul respectif d'une valeur « Indice Infestation Insecte » dénommée
« 3I » (ou « indice 3I ») et d'une valeur « Valeur limite » dénommée « VL », qui sont dépendantes chacune de la présence ou de l'absence des COV cibles prédéterminés,
e/ la comparaison des valeurs « 3I » et « VL », si la valeur « 3I » est strictement supérieure à la valeur « VL » alors l'environnement est infesté, et si la valeur « 3I » est inférieure ou égale à la valeur « VL » alors l'environnement n'est pas infesté.

Conformément au procédé de l'invention, l'étape a/ de prélèvement de l'échantillon de COV est de préférence réalisée par échantillonnage actif ou diffusif sur un adsorbant solide préférentiellement choisi parmi le charbon actif, le gel de silice, les zéolithes et les résines synthétiques poreuses, tels ceux commercialisés sous la marque Tenax®, Carbograph® ou Chromosorb®.

Dans ce cas, le procédé de l'invention comprend également une étape de désorption des COV adsorbés. Celle-ci est effectuée par thermo-désorption dans des conditions bien connues de l'homme du métier.

Le procédé de l'invention comprend également la séparation des COV prélevés (étape b/). En particulier, les COV prélevés sont séparés par élution sur une micro-colonne chromatographique. Les paramètres optimums de séparation comme la température de la colonne ou le débit de la phase mobile, sont déterminés selon les techniques bien connues de l'homme du métier en fonction de la géométrie de la colonne, de la nature de la phase stationnaire et du gaz vecteur.

L'étape c/ de détection permet de détecter la présence ou l'absence de COV « cibles » prédéterminés, issus du métabolisme des insectes, y compris à l'état de larves. Ces COV « cibles » prédéterminés, en lien avec l'activité des insectes, y compris à l'état de larves, comprennent au moins un COV de l'une des catégories 1, 2 ou 3.

En détectant la présence ou l'absence d'au moins un COV cible prédéterminé de chacune des trois catégories de COV citées ci-dessus, on augmente la certitude du procédé de détection selon l'invention par rapport à une détection de la présence ou l'absence de COV issus d'une seule de ces catégories.

De préférence, on détectera plusieurs COV cibles de chacune des trois catégories précitées.

Les COV cibles prédéterminés sont par exemple détectés par chromatographie en phase gazeuse suivie de spectrométrie de masse (GC/MS).

La prochaine étape d/ du procédé de l'invention consiste ensuite à calculer la valeur « Indice Infestation Insecte » (« 3I » ou indice 3I) et la valeur « Valeur limite » (« VL »).

Plus particulièrement, selon l'invention, l'étape d/ est réalisée en trois sous-étapes :

d-1/ détermination d'une valeur d'incrémentation « i », la valeur d'incrémentation étant obtenue par attribution à chacun des COV cibles pré-déterminés d'un chiffre de -1, 0 ou 1 en fonction de sa présence ou de son absence, l'attribution des chiffres se faisant de la façon suivante :

- la présence de COV de la catégorie (1) est caractérisée par le chiffre 1 et son absence par -1 ;
- la présence de COV de la catégorie (2) est caractérisée par le chiffre 0 et son absence par -1 ;
- la présence de COV de la catégorie (3) est caractérisée par le chiffre 1 et son absence par 0 ;

d-2/ détermination d'une valeur de pondération « P » (encore dénommée « facteur de pondération ») correspondant à la formule suivante :

$$P = \frac{\text{Coefficient 1}}{\text{Nombre de "COV pondérés"}}$$

dans laquelle :

- les « COV pondérés » correspondent aux COV retrouvés sur des supports sans infestation active mais émis en quantité relative plus importante en présence des insectes, à savoir en une quantité au moins deux fois supérieure,
- le coefficient 1 est un nombre réel empirique fixé par itération avec un panel d'environnements dont l'infestation est connue ;

**d-3/** détermination de la valeur 3I et VL,

- la valeur (3I) correspondant à la formule suivante :

$$(3I) = \sum_{j=n}^{1} i_j \times P_j$$

dans laquelle :

n représente le nombre de COV cibles,
j représente un nombre entier,
i représente la valeur d'incrémentation telle que définie à l'étape d-1/,
P représente la valeur de pondération telle que définie à l'étape d-2/,
$i_j$ représente l'incrémentation du $j^{ème}$ COV cible,
$P_j$ représente la pondération du $j^{ème}$ COV cible,

- la valeur (VL) correspondant à la formule suivante :

$$VL = \frac{\text{Nombre de "COV non pondérés"} + P \times \text{Nombre de "COV pondérés"}}{\text{Coefficient 2}}$$

dans laquelle les « COV non pondérés » correspondent aux COV émis uniquement en présence d'insectes,
le coefficient 2 est un nombre réel empirique fixé par itération avec le même panel d'environnement que celui utilisé pour la détermination du « coefficient 1 ».

Le coefficient 1 est donc fixé pour permettre la discrimination des deux groupes du panel (infesté/non infesté). Ce coefficient est fonction de l'espèce d'insecte à détecter.
De même, le coefficient 2 est fixé pour permettre la discrimination des deux groupe du panel (infesté/non infesté). Ce coefficient est fonction de l'espèce d'insecte à détecter.
Dans un premier temps (étape **d-1/**) il s'agit de déterminer une valeur d'incrémentation « i » qui est fonction de la présence ou de l'absence de chaque COV cible prédéterminé.
Plus particulièrement, lors de ses recherches la Société Demanderesse a pu faire les constatations ci-après.
La présence de COV spécifiquement issus du métabolisme des insectes, y compris à l'état de larves, qui sont émis indépendamment du support sur lequel les insectes se développent et qui ne sont émis que lors d'une infestation par des insectes (COV de la catégorie 1), indique une infestation par des insectes tandis que l'absence de tels COV indique l'absence d'une infestation par des insectes.
Par contre, la présence de COV spécifiquement issus du métabolisme des insectes, y compris à l'état de larves, qui sont émis indépendamment du support sur lequel les insectes se développent et qui peuvent aussi avoir d'autres origines biologiques que les insectes (COV de la catégorie 2) ne permet pas de conclure à une infestation par des insectes. Toutefois, l'absence de tels COV indique l'absence d'une infestation par des insectes.
En ce qui concerne les COV spécifiquement issus du métabolisme des insectes, y compris à l'état de larves, qui sont émis en fonction du support sur lequel les insectes se développent et qui ne sont émis que lors d'une infestation par des insectes (COV de la catégorie 3), leur présence indique une infestation par des insectes tandis que leur absence ne permet pas de conclure sur l'absence d'une infestation par des insectes.

Sur la base de ces constatations, une méthode de calcul a été mise au point par les Inventeurs qui est basée sur l'incrémentation suivante.

La présence d'un COV est incrémentée d'une valeur « 1 » si la présence du COV indique une infestation par des insectes et d'une valeur « 0 » si la présence du COV ne permet pas de conclure à la présence d'une infestation par des insectes. L'absence d'un COV est incrémentée d'une valeur « -1 » si l'absence du COV indique l'absence d'une infestation par des insectes et d'une valeur « 0 » si l'absence du COV ne permet pas de conclure à l'absence infestation par des insectes.

Le tableau 1 ci-dessous résume les principes d'incrémentation.

**Tableau 1:** Incrémentation "i"

| COV | Incrémentation « i » | |
|---|---|---|
| | présence | absence |
| émis indépendamment du support, émis que lors d'une infestation par des insectes (catégorie 1) | 1 | -1 |
| émis indépendamment du support, peut également avoir d'autres origines biologiques (catégorie 2) | 0 | -1 |
| émis en fonction du support, émis que lors d'une infestation par des insectes (catégorie 3) | 1 | 0 |

Cependant cette première étape **d-1**/ ne permet pas à elle seule de discriminer des environnements infestés d'environnements « sains ».

Par conséquent, afin de permettre cette discrimination, une deuxième étape **d-2**/ qui consiste en une pondération, permet de compléter cette première étape.

Les COV retrouvés en laboratoire en contexte de référence (support sans infestation active), mais émis en quantité relative plus importante en présence des insectes (quantité au moins 2 fois supérieure), sont ici pris en compte et pondérés par un facteur appelé « P » défini comme suit :

$$P = \frac{\text{Coefficient 1}}{\text{Nombre de "COV pondérés"}}$$

Ces COV sont nommés « COV pondérés ». Cette pondération a pour objectif de réduire l'impact de ces composés (« COV pondérés ») vis-à-vis de composés émis uniquement par des insectes (« COV non pondérés ») au laboratoire.

Ainsi, la détection de n « COV pondérés » (n = Nombre de « COV pondérés » = Coefficient 1/P), équivaut à 1 « COV non pondéré ».

Le « coefficient 1 » est un nombre réel empirique déterminé expérimentalement avec les mesures *in situ*. Ce coefficient a été fixé par itération avec un panel d'environnements dont l'infestation est connue (infesté/non infesté).

Cette pondération P est égale à 1 (P = 1) dans le cas des COV émis uniquement en présence d'insectes « COV non pondérés ». Cette pondération unitaire permet de donner la même importance à chaque « COV non pondéré ».

Enfin, la troisième étape d-**3**/ permet de déterminer respectivement les valeurs « 3I » (indice 3I) et « VL ».

L'indice 3I s'incrémente de la manière suivante :

$$(3I) = \sum_{j=n}^{1} i_j \times P_j$$

avec n, j, i, P, $i_j$, $P_j$ tels que définis ci-dessus, à savoir :

n représente le nombre de COV cibles,

j représente un nombre entier,

i représente la valeur d'incrémentation telle que définie à l'étape e1/,

P représente la valeur de pondération telle que définie à l'étape e2/,

$i_j$ représente l'incrémentation du j$^{\text{ème}}$ COV cible,

$P_j$ représente la pondération du j$^{\text{ème}}$ COV cible,

La valeur (VL) correspond à la formule déjà décrite, à savoir :

$$VL = \frac{\text{Nombre de "COV non pondérés"} + P \times \text{Nombre de "COV pondérés"}}{\text{Coefficient 2}}$$

Le « coefficient 2 » est un nombre réel empirique déterminé expérimentalement avec les mesures *in situ*. Comme précédemment pour le « coefficient 1 », le « coefficient 2 » a été fixé par itération avec le même panel d'environnements qui a été utilisé pour la détermination du « coefficient 1 ».

Ces coefficients « 1 » et « 2 » permettent, par une approche empirique, de discriminer deux groupes distincts (infestés et non infestés) parmi un panel d'environnements dont l'état d'infestation est connu.

Si l'indice (3I) est strictement supérieur à VL (3I > VL), alors l'environnement est infesté, dans le cas contraire, si l'indice (3I) est inférieur ou égal à VL (3I ≤ VL) il n'est pas infesté.

Selon l'invention, les COV cibles prédéterminés sont de préférence choisis dans le groupe comprenant le 2,2'-bi-1,3-dioxolane ; l'acétate de n-butyle ; le 1,3,5,7-cyclooctatetraene ; l'alpha-pinène ; le 1-propanol ; l'acide méthane carbothiolique ; le 3,3-dimethyl-1-hexene ; le 2-butanone ; l'acétate d'éthyle ; le 4-hydroxy-2-butanone ; le 1-butanol ; le 2,5-diméthyl-furane ; le 2-éthényl-2-buténal ; le butyle formate ; la pyrazine ; le pyrrole ; le 1-pentanol ; le 1-(3,4-diméthylthiéno[2,3-b]thiophèn-2-yl)-éthanoneoxime ; le 1,4-octadiène ; le 1,3-octadiène ; le 2-méthyl-pyrimidine ; le 1-hexanol ; le 2-heptanone ; le 2,5-diméthyl-pyrazine ; le camphène ; le 2-octanone ; le 1-méthyl-4-(1-méthyléthényl)-cyclohéxène ; le 2-méthyl-décane ; l'acétophénone ; le 2-nonèn-1-ol ; le 1,10-dichlorodécane ; le dodécanal ; le 2-pentyl-thiophène ; le 2-decène-1-ol ; le naphtalène ; le diéthyl phthalate, et leurs mélanges.

Plus particulièrement, selon un mode de réalisation avantageux de l'invention :

- les COV cibles prédéterminés de la catégorie 1 sont choisis dans le groupe comprenant le 2,2'-bi-1,3-dioxolane et l'acétate de n-butyle ;
- les COV cibles prédéterminés de la catégorie 2 sont choisis dans le groupe comprenant le 1,3,5,7-cyclooctatetraene et l'alpha-pinène ;
- les COV cibles prédéterminés de la catégorie 3 sont choisis dans le groupe comprenant le 1-propanol ; l'acide méthane carbothiolique ; le 3,3-dimethyl-1-hexene ; le 2-butanone ; l'acétate d'éthyle ; le 4-hydroxy-2-butanone ; le 1-butanol ; le 2,5-diméthyl-furane ; le 2-éthényl-2-buténal ; le butyle formate ; la pyrazine ; le pyrrole ; le 1-pentanol ; le 1-(3,4-diméthylthiéno[2,3-b]thiophèn-2-yl)-éthanoneoxime ; le 1,4-octadiène; le 1,3-octadiène ; le 2-méthyl-pyrimidine ; le 1-hexanol ; le 2-heptanone ; le 2,5-diméthyl-pyrazine ; le camphène ; le 2-octanone ; le 1-méthyl-4-(1-méthyléthényl)-cyclohéxène ; le 2-méthyl-décane ; l'acétophénone ; le 2-nonèn-1-ol ; le 1,10-dichlorodécane ; le dodécanal ; le 2-pentyl-thiophène ; le 2-decène-1-ol ; le naphtalène et le diéthyl phthalate.

Le procédé de détection d'une infestation d'insectes selon l'invention est particulièrement utile pour la détection précoce d'une telle infestation, c'est-à-dire avant l'apparition de signes visibles.

L'invention a encore pour objet un procédé de détection de la présence d'insectes tel que défini ci-dessus, caractérisé en ce que les insectes sont à l'état de larves.

L'exemple de réalisation suivant illustre la présente invention, sans en limiter en aucune façon la portée.

## EXEMPLE

L'objectif des travaux ci-dessous présentés est de déterminer, dans une première phase laboratoire, une liste de COV cibles émis durant l'infestation de supports par un insecte au stade larvaire. Dans la pratique, des supports infestés et non infestés seront placés dans des chambres d'émission avant prélèvements des COV.

Dans la seconde phase, les cibles COV seront recherchées dans des prélèvements réalisés dans des environnements intérieurs présentant ou non une infestation par ces insectes.

**MATERIEL ET METHODES**

**Matériel biologique**

L'espèce d'insectes retenue est un microlépidoptère kératophage, la mite des vêtements, *Tineola bisselliella*. Durant la phase laboratoire, deux à trois larves de cette espèce issues d'un élevage ont été déposées dans les chambres dites « chambres infestées ».

Selon un mode de réalisation du procédé de détection de l'invention, lorsque l'insecte est *Tineola bisselliella*, alors :

- le coefficient 1 présente une valeur allant de 1 à 3, et est de préférence égal à 2,
- le coefficient 2 présente une valeur allant de 3 à 5, et est de préférence égal à 4.

## Support de développement

Deux types de supports, vulnérables vis-à-vis des mites, sont employés durant la phase laboratoire :

- une pièce de laine tissée,
- de la peau de lapin.

## Chambre d'émission

Des chambres d'émission de 300 cm3, développées par CSTB, sont employées durant la phase laboratoire. Elles sont en verre et dotées de vannes PTFE pour réaliser les prélèvements COV.

Pour chaque série d'analyses, une chambre de référence, exempte de mites et contenant uniquement le substrat nourricier, à savoir de la laine ou de la peau de lapin a été utilisée. Les chambres d'émissions sont ensuite incubées dans une étuve d'élevage à une température de 25°C et à l'abri de la lumière. La durée de l'incubation varie selon les expériences réalisées, elle est comprise entre 3 et 6 mois.

## Environnements in situ

Trois types d'environnement ont été investigués. Un diagnostic visuel et un autre couplé à l'emploi de pièges à phéromones, a été réalisé pour déterminer la présence ou l'absence de mites des vêtements dans les différents lieux examinés. Il s'agit d'un palais situé en région Ile-de-France (2 salles), de deux logements et d'un musée (dans une voiture hippomobile infestée et dans le hangar).

Le tableau 2 décrit les environnements investigués et leur état d'infestation.

**Tableau 2** : environnements investigués et état d'infestation

| Type | Lieu | Etat d'infestation |
|---|---|---|
| Palais | Palais 1<br>Palais 2 | Infesté<br>Infesté |
| Musée | Voiture int<br>Voiture ext | Infesté<br>Non infesté |
| Logement | Logement 1<br>Logement 2 | Non infesté<br>Non infesté |

## Prélèvement

Les COV sont collectés sur des tubes contenant un adsorbant, le TENAX®TA (Sigma Aldrich). Il s'agit d'une phase apolaire, constituée d'un polymère adsorbant de 2,6-diphényl-p-phénylène oxyde. Celui-ci permet de retenir les molécules dont le nombre de carbone est compris entre $C_4$ et $C_{20}$. Le prélèvement est actif, il est réalisé à l'aide d'une pompe. Le débit de prélèvement est fixé à 100 cm$^3$/min et le temps de prélèvement est suffisant pour renouveler à minima dix fois le volume de la chambre.

Dans le cas des prélèvements environnementaux réalisés dans les espaces clos, le débit est de 150 cm$^3$/min et la durée du prélèvement est d'une heure.

## Analyse

La désorption des tubes de TENAX®TA est réalisée sur un ATD 400, Perkin Elmer. Les COV sont injectés simultanément sur une colonne de type VF-5ms (Agilent), ils sont ensuite séparés et analysés avec un système GC-MS (GC 3800 - MS Saturn 2000, Varian). Les analytes sont identifiés par leur rétention et par comparaison de leur spectre de masse avec la bibliothèque NIST 2008. L'identification des composés est confirmée par le passage d'un standard, selon disponibilité.

**RESULTATS**

**Phase laboratoire**

Cette phase permet d'identifier une liste de COV cibles émis lors de l'infestation de supports par des insectes. Chaque COV cible a ensuite été catégorisé dans un des trois «catégorie 1, 2 ou 3» comme décrit dans la présente demande (classification des COV produits selon leur spécificité).

La présence ou l'absence du COV cible (« catégorie 1, 2 ou 3») dans le contexte de référence a permis de déterminer une incrémentation « i » puis la pondération P, et enfin l'indice 3I.

Dans le cas présent on obtient respectivement :

- une pondération P égale à 1 pour les « COV non pondérés »,
- une pondération P égale à 0,08 pour les « COV pondérés ».

La pondération P inférieure à 1 (P =0,08) permet de limiter l'impact d'un « COV pondéré ». Ainsi, plusieurs COV pondérés sont nécessaires pour impacter de la même manière qu'un « COV non pondéré ».

Les résultats obtenus sont présentés dans le tableau 3.

Concernant la colonne « Présence/Absence », le chiffre 0 indique l'absence des COV et le chiffre 1 indique la présence des COV.

Concernant la colonne « Incrémentation », les chiffres 1, 0 et -1 sont tels que décrits dans le tableau 1.

**Tableau 3** : Emission des COV sur des supports non infestés, anciennement infestés et infestés

| | Présence/Absence | | | | | | | | | | Catégorie de COV | Incrémentation si présence | Incrémentation si absence | Pondération (P) | i x P si présence | i x P si absence | Incrémentation (i) | | | | | | | | | |
|---|---|---|---|---|---|---|---|---|---|---|---|---|---|---|---|---|---|---|---|---|---|---|---|---|---|---|
| | Laine (référence 1) non infestée | Laine (référence 2) non infestée | Peau de lapin (référence) non infestée | Laine anciennement infestée 1 | Laine anciennement infestée 2 | Laine infestée 1 | Laine infestée 2 | Laine infestée 3 | Laine infestée 4 | Peau de lapin infestée | | | | | | | Laine (référence 1) | Laine (référence 2) | Peau de lapin (référence) | Laine anciennement infestée 1 | Laine anciennement infestée 2 | Laine infestée 1 | Laine infestée 2 | Laine infestée 3 | Laine infestée 4 | Peau de lapin infestée |
| 1-propanol | 0 | 0 | 1 | 0 | 0 | 0 | 1 | 1 | 0 | 1 | 3 | 1 | 0 | 0.08 | 0.08 | 0 | 0 | 0 | 0.08 | 0 | 0 | 0 | 0.08 | 0.08 | 0 | 0.08 |
| Acide methanecarbothiolique | 0 | 1 | 0 | 0 | 1 | 1 | 0 | 1 | 1 | 0 | 3 | 1 | 0 | 0.08 | 0.08 | 0 | 0 | 0.08 | 0 | 0 | 0.08 | 0.08 | 0 | 0.08 | 0.08 | 0 |
| 3,3-dimethyl-1-Hexene | 0 | 0 | 0 | 0 | 0 | 1 | 0 | 0 | 0 | 0 | 3 | 1 | 0 | 0.08 | 0.08 | 0 | 0 | 0 | 0 | 0 | 0 | 0.08 | 0 | 0 | 0 | 0 |
| 2-Butanone | 0 | 0 | 1 | 0 | 0 | 0 | 0 | 0 | 1 | 1 | 3 | 1 | 0 | 0.08 | 0.08 | 0 | 0 | 0 | 0.08 | 0 | 0 | 0 | 0 | 0 | 0.08 | 0.08 |
| Ethyl Acetate | 0 | 0 | 0 | 0 | 1 | 0 | 1 | 0 | 1 | 0 | 3 | 1 | 0 | 1 | 1 | 0 | 0 | 0 | 0 | 0 | 1 | 0 | 1 | 0 | 1 | 0 |
| 4-hydroxy-2-Butanone | 0 | 0 | 1 | 0 | 0 | 0 | 0 | 0 | 0 | 1 | 3 | 1 | 0 | 0.08 | 0.08 | 0 | 0 | 0 | 0.08 | 0 | 0 | 0 | 0 | 0 | 0 | 0.08 |
| 2,2'-Bi-1,3-dioxolane | 1 | 0 | 0 | 0 | 0 | 1 | 1 | 1 | 1 | 1 | 1 | 1 | -1 | 1 | 1 | -1 | 1 | -1 | -1 | -1 | -1 | 1 | 1 | 1 | 1 | 1 |
| 1-Butanol | 0 | 1 | 1 | 0 | 1 | 0 | 1 | 1 | 1 | 1 | 3 | 1 | 0 | 0.08 | 0.08 | 0 | 0 | 0.08 | 0.08 | 0 | 0.08 | 0 | 0.08 | 0.08 | 0.08 | 0.08 |
| 2,5-dimethyl-Furan | 0 | 1 | 0 | 0 | 1 | 0 | 1 | 1 | 1 | 1 | 3 | 1 | 0 | 0.08 | 0.08 | 0 | 0 | 0.08 | 0 | 0 | 0.08 | 0 | 0.08 | 0.08 | 0.08 | 0.08 |
| 2-ethenyl-2-Butenal | 0 | 0 | 1 | 0 | 1 | 0 | 0 | 1 | 1 | 1 | 3 | 1 | 0 | 0.08 | 0.08 | 0 | 0 | 0 | 0.08 | 0 | 0.08 | 0 | 0 | 0.08 | 0.08 | 0.08 |
| Butyl formate | 0 | 1 | 0 | 0 | 0 | 0 | 1 | 0 | 1 | 0 | 3 | 1 | 0 | 0.08 | 0.08 | 0 | 0 | 0.08 | 0 | 0 | 0 | 0 | 0.08 | 0 | 0.08 | 0 |
| Pyrazine | 0 | 0 | 0 | 0 | 0 | 0 | 0 | 1 | 0 | 1 | 3 | 1 | 0 | 1 | 1 | 0 | 0 | 0 | 0 | 0 | 0 | 0 | 0 | 1 | 0 | 1 |
| Pyrrole | 0 | 0 | 0 | 0 | 0 | 0 | 1 | 0 | 0 | 1 | 3 | 1 | 0 | 1 | 1 | 0 | 0 | 0 | 0 | 0 | 0 | 0 | 1 | 0 | 0 | 1 |
| 1-Pentanol | 0 | 0 | 1 | 1 | 1 | 0 | 0 | 0 | 1 | 1 | 3 | 1 | 0 | 0.08 | 0.08 | 0 | 0 | 0 | 0.08 | 0.08 | 0.08 | 0 | 0 | 0 | 0.08 | 0.08 |
| 1-(3,4-dimethylthieno[2,3-b]thiophen-2-yl)-Ethanoneoxime | 0 | 0 | 0 | 0 | 0 | 1 | 0 | 0 | 0 | 0 | 3 | 1 | 0 | 1 | 1 | 0 | 0 | 0 | 0 | 0 | 0 | 1 | 0 | 0 | 0 | 0 |
| Acétate de n-butyle | 0 | 0 | 1 | 0 | 1 | 0 | 1 | 0 | 1 | 1 | 1 | 1 | -1 | 0.08 | 0.08 | -0.08 | -0.08 | -0.08 | 0.08 | -0.08 | 0.08 | -0.08 | 0.08 | -0.08 | 0.08 | 0.08 |
| 1,4-Octadiene | 0 | 0 | 0 | 0 | 0 | 0 | 1 | 0 | 1 | 0 | 3 | 1 | 0 | 1 | 1 | 0 | 0 | 0 | 0 | 0 | 0 | 0 | 1 | 0 | 1 | 0 |
| 1,3-Octadiene | 0 | 0 | 1 | 0 | 0 | 0 | 0 | 0 | 0 | 1 | 3 | 1 | 0 | 0.08 | 0.08 | 0 | 0 | 0 | 0.08 | 0 | 0 | 0 | 0 | 0 | 0 | 0.08 |
| 2-methyl-Pyrimidine | 0 | 0 | 0 | 0 | 0 | 0 | 0 | 1 | 0 | 1 | 3 | 1 | 0 | 1 | 1 | 0 | 0 | 0 | 0 | 0 | 0 | 0 | 0 | 1 | 0 | 1 |
| 1-Hexanol | 0 | 0 | 1 | 0 | 0 | 0 | 0 | 1 | 1 | 1 | 3 | 1 | 0 | 0.08 | 0.08 | 0 | 0 | 0 | 0.08 | 0 | 0 | 0 | 0 | 0.08 | 0.08 | 0.08 |
| 2-Heptanone | 1 | 0 | 1 | 0 | 1 | 0 | 0 | 0 | 1 | 1 | 3 | 1 | 0 | 0.08 | 0.08 | 0 | 0.08 | 0 | 0.08 | 0 | 0.08 | 0 | 0 | 0 | 0.08 | 0.08 |
| 1,3,5,7-Cyclooctatetraene | 1 | 1 | 1 | 1 | 1 | 1 | 1 | 0 | 1 | 1 | 2 | 0 | -1 | 0.08 | 0 | -0.08 | 0 | 0 | 0 | 0 | 0 | 0 | 0 | -0.08 | 0 | 0 |
| 2,5-dimethyl-Pyrazine | 0 | 0 | 0 | 0 | 0 | 1 | 0 | 0 | 0 | 1 | 3 | 1 | 0 | 1 | 1 | 0 | 0 | 0 | 0 | 0 | 0 | 1 | 0 | 0 | 0 | 1 |
| α-pinene | 0 | 1 | 1 | 1 | 1 | 1 | 1 | 1 | 1 | 1 | 2 | 0 | -1 | 0.08 | 0 | -0.08 | -0.08 | 0 | 0 | 0 | 0 | 0 | 0 | 0 | 0 | 0 |
| Camphene | 0 | 0 | 0 | 0 | 1 | 0 | 1 | 0 | 1 | 0 | 3 | 1 | 0 | 1 | 1 | 0 | 0 | 0 | 0 | 0 | 1 | 0 | 1 | 0 | 1 | 0 |
| 2-Octanone | 1 | 0 | 1 | 0 | 0 | 0 | 1 | 1 | 1 | 1 | 3 | 1 | 0 | 0.08 | 0.08 | 0 | 0.08 | 0 | 0.08 | 0 | 0 | 0 | 0.08 | 0.08 | 0.08 | 0.08 |
| 1-methyl-4-(1-methylethenyl)-Cyclohexene | 1 | 1 | 1 | 0 | 0 | 0 | 1 | 1 | 1 | 1 | 3 | 1 | 0 | 0.08 | 0.08 | 0 | 0.08 | 0.08 | 0.08 | 0 | 0 | 0 | 0.08 | 0.08 | 0.08 | 0.08 |
| 2-methyl-Decane | 0 | 0 | 0 | 0 | 0 | 0 | 0 | 0 | 0 | 1 | 3 | 1 | 0 | 1 | 1 | 0 | 0 | 0 | 0 | 0 | 0 | 0 | 0 | 0 | 0 | 1 |
| Acetophenone | 1 | 1 | 1 | 1 | 1 | 1 | 0 | 0 | 0 | 1 | 3 | 1 | 0 | 0.08 | 0.08 | 0 | 0.08 | 0.08 | 0.08 | 0.08 | 0.08 | 0.08 | 0 | 0 | 0 | 0.08 |
| 2-Nonen-1-ol | 1 | 1 | 1 | 1 | 1 | 1 | 1 | 1 | 1 | 1 | 3 | 1 | 0 | 1 | 1 | 0 | 1 | 1 | 1 | 1 | 1 | 1 | 1 | 1 | 1 | 1 |
| 1,10-Dichlorodecane | 1 | 0 | 0 | 0 | 0 | 0 | 1 | 1 | 1 | 0 | 3 | 1 | 0 | 0.08 | 0.08 | 0 | 0.08 | 0 | 0 | 0 | 0.08 | 0 | 0.08 | 0.08 | 0.08 | 0 |
| Dodecanal | 0 | 0 | 0 | 0 | 0 | 0 | 1 | 0 | 1 | 0 | 3 | 1 | 0 | 1 | 1 | 0 | 0 | 0 | 0 | 0 | 0 | 0 | 1 | 0 | 1 | 0 |
| 2-pentyl-Thiophene | 0 | 0 | 0 | 0 | 0 | 0 | 0 | 0 | 0 | 1 | 3 | 1 | 0 | 1 | 1 | 0 | 0 | 0 | 0 | 0 | 0 | 0 | 0 | 0 | 0 | 1 |
| 2-Decen-1-ol | 0 | 1 | 1 | 1 | 1 | 0 | 1 | 1 | 0 | 0 | 3 | 1 | 0 | 0.08 | 0.08 | 0 | 0 | 0.08 | 0.08 | 0.08 | 0.08 | 0 | 0.08 | 0.08 | 0 | 0 |
| Naphthalene | 1 | 1 | 1 | 1 | 1 | 1 | 1 | 1 | 1 | 1 | 3 | 1 | 0 | 0.08 | 0.08 | 0 | 0.08 | 0.08 | 0.08 | 0.08 | 0.08 | 0.08 | 0.08 | 0.08 | 0.08 | 0.08 |
| Diethyl Phthalate | 0 | 1 | 1 | 0 | 1 | 0 | 1 | 1 | 1 | 1 | 3 | 1 | 0 | 0.08 | 0.08 | 0 | 0 | 0.08 | 0.08 | 0 | 0.08 | 0 | 0.08 | 0.08 | 0.08 | 0.08 |
| Indice 3I | | | | | | | | | | | | | | | | | 2.32 | 0.64 | 1.28 | 0.24 | 2.96 | 4.24 | 7.88 | 4.8 | 7.2 | 9.28 |

Comme déjà indiqué, pour la valeur (3I) dédiée à *Tineola bisselliella*, les coefficients 1 et 2 sont fixés respectivement à 2 et 4.

**Phase in situ**

Cette phase permet d'une part de confronter la liste de COV cibles identifiés dans la phase laboratoire avec plusieurs bruits de fond rencontrés en environnements réels. D'autre part, elle permet de comparer les émissions dans des environnements infestés et non infestés.
Les résultats sont présentés dans le tableau 4.

## Tableau 4 : émission COV d'environnements infestés et non infestés

| | Présence/absence | | | | | | | Incrémentation (i) | | | | | | | | | | |
| --- | --- | --- | --- | --- | --- | --- | --- | --- | --- | --- | --- | --- | --- | --- | --- | --- | --- | --- |
| | Voiture ext | Logement 1 | Logement 2 | Voiture int | Palais 1 | Palais 2 | Catégorie de COV | Incrémentation si présence | Incrémentation si absence | Pondération (P) | i x P si présence | i x P si absence | Voiture ext | Logement 1 | Logement 2 | Voiture int | Palais 1 | Palais 2 |
| 1-propanol | 1 | 0 | 0 | 0 | 1 | 1 | 3 | 1 | 0 | 0.08 | 0.08 | 0 | 0.08 | 0 | 0 | 0 | 0.08 | 0.08 |
| Acide methanecarbothiolique | 0 | 1 | 1 | 1 | 1 | 1 | 3 | 1 | 0 | 0.08 | 0.08 | 0 | 0 | 0.08 | 0.08 | 0.08 | 0.08 | 0.08 |
| 3,3-dimethyl-1-Hexene | 0 | 0 | 0 | 0 | 0 | 0 | 3 | 1 | 0 | 0.08 | 0.08 | 0 | 0 | 0 | 0 | 0 | 0 | 0 |
| 2-Butanone | 1 | 1 | 0 | 1 | 1 | 1 | 3 | 1 | 0 | 0.08 | 0.08 | 0 | 0.08 | 0.08 | 0 | 0.08 | 0.08 | 0.08 |
| Ethyl Acetate | 1 | 0 | 0 | 0 | 1 | 1 | 3 | 1 | 0 | 1 | 1 | 0 | 1 | 0 | 0 | 0 | 1 | 1 |
| 4-hydroxy-2-Butanone | 1 | 0 | 0 | 1 | 0 | 0 | 3 | 1 | 0 | 0.08 | 0.08 | 0 | 0.08 | 0 | 0 | 0.08 | 0 | 0 |
| 2,2'-Bi-1,3-dioxolane | 1 | 0 | 0 | 1 | 1 | 1 | 1 | 1 | -1 | 1 | 1 | -1 | 1 | -1 | -1 | 1 | 1 | 1 |
| 1-Butanol | 1 | 0 | 1 | 0 | 0 | 1 | 3 | 1 | 0 | 0.08 | 0.08 | 0 | 0.08 | 0 | 0.08 | 0 | 0 | 0.08 |
| 2,5-dimethyl-Furan | 0 | 0 | 0 | 0 | 0 | 0 | 3 | 1 | 0 | 0.08 | 0.08 | 0 | 0 | 0 | 0 | 0 | 0 | 0 |
| 2-ethenyl-2-Butenal | 0 | 0 | 0 | 0 | 0 | 0 | 3 | 1 | 0 | 0.08 | 0.08 | 0 | 0 | 0 | 0 | 0 | 0 | 0 |
| Butyl formate | 0 | 0 | 0 | 0 | 0 | 0 | 3 | 1 | 0 | 0.08 | 0.08 | 0 | 0 | 0 | 0 | 0 | 0 | 0 |
| Pyrazine | 0 | 1 | 1 | 0 | 0 | 0 | 3 | 1 | 0 | 1 | 1 | 0 | 0 | 1 | 1 | 0 | 0 | 0 |
| Pyrrole | 0 | 0 | 1 | 0 | 1 | 0 | 3 | 1 | 0 | 1 | 1 | 0 | 0 | 0 | 1 | 0 | 1 | 0 |
| 1-Pentanol | 1 | 0 | 0 | 1 | 1 | 1 | 3 | 1 | 0 | 0.08 | 0.08 | 0 | 0.08 | 0 | 0 | 0.08 | 0.08 | 0.08 |
| 1-(3,4-dimethylthieno[2,3-b]thiophen-2-yl)-Ethanoneoxime | 0 | 0 | 0 | 1 | 0 | 1 | 3 | 1 | 0 | 1 | 1 | 0 | 0 | 0 | 0 | 1 | 0 | 1 |
| Acétate de n-butyle | 1 | 0 | 1 | 1 | 1 | 0 | 1 | 1 | -1 | 0.08 | 0.08 | -0.08 | 0.08 | -0.08 | 0.08 | 0.08 | 0.08 | -0.08 |
| 1,4-Octadiene | 0 | 0 | 0 | 0 | 0 | 0 | 3 | 1 | 0 | 1 | 1 | 0 | 0 | 0 | 0 | 0 | 0 | 0 |
| 1,3-Octadiene | 0 | 0 | 1 | 0 | 0 | 0 | 3 | 1 | 0 | 0.08 | 0.08 | 0 | 0 | 0 | 0.08 | 0 | 0 | 0 |
| 2-methyl-Pyrimidine | 0 | 0 | 0 | 0 | 0 | 0 | 3 | 1 | 0 | 1 | 1 | 0 | 0 | 0 | 0 | 0 | 0 | 0 |
| 1-Hexanol | 0 | 0 | 0 | 0 | 0 | 0 | 3 | 1 | 0 | 0.08 | 0.08 | 0 | 0 | 0 | 0 | 0 | 0 | 0 |
| 2-Heptanone | 0 | 1 | 0 | 1 | 0 | 0 | 3 | 1 | 0 | 0.08 | 0.08 | 0 | 0 | 0.08 | 0 | 0.08 | 0 | 0 |
| 1,3,5,7-Cyclooctatetraene | 1 | 0 | 0 | 1 | 1 | 1 | 2 | 0 | -1 | 0.08 | 0 | -0.08 | 0 | -0.08 | -0.08 | 0 | 0 | 0 |
| 2,5-dimethyl-Pyrazine | 0 | 0 | 0 | 0 | 0 | 0 | 3 | 1 | 0 | 1 | 1 | 0 | 0 | 0 | 0 | 0 | 0 | 0 |
| α-pinene | 1 | 1 | 1 | 1 | 1 | 1 | 2 | 0 | -1 | 0.08 | 0 | -0.08 | 0 | 0 | 0 | 0 | 0 | 0 |
| Camphene | 0 | 0 | 0 | 0 | 0 | 0 | 3 | 1 | 0 | 1 | 1 | 0 | 0 | 0 | 0 | 0 | 0 | 0 |
| 2-Octanone | 0 | 1 | 0 | 0 | 0 | 0 | 3 | 1 | 0 | 0.08 | 0.08 | 0 | 0 | 0.08 | 0 | 0 | 0 | 0 |
| 1-methyl-4-(1-methylethenyl)-Cyclohexene | 0 | 1 | 0 | 0 | 1 | 0 | 3 | 1 | 0 | 0.08 | 0.08 | 0 | 0 | 0.08 | 0 | 0 | 0.08 | 0 |
| 2-methyl-Decane | 0 | 0 | 1 | 0 | 0 | 1 | 3 | 1 | 0 | 1 | 1 | 0 | 0 | 0 | 1 | 0 | 0 | 1 |
| Acetophenone | 1 | 1 | 1 | 1 | 0 | 1 | 3 | 1 | 0 | 0.08 | 0.08 | 0 | 0.08 | 0.08 | 0.08 | 0.08 | 0 | 0.08 |
| 2-Nonen-1-ol | 0 | 0 | 0 | 1 | 0 | 1 | 3 | 1 | 0 | 1 | 1 | 0 | 0 | 0 | 0 | 1 | 0 | 1 |
| 1,10-Dichlorodecane | 0 | 0 | 0 | 0 | 0 | 0 | 3 | 1 | 0 | 0.08 | 0.08 | 0 | 0 | 0 | 0 | 0 | 0 | 0 |
| Dodecanal | 0 | 0 | 0 | 0 | 1 | 0 | 3 | 1 | 0 | 1 | 1 | 0 | 0 | 0 | 0 | 0 | 1 | 0 |
| 2-pentyl-Thiophene | 0 | 0 | 0 | 0 | 0 | 0 | 3 | 1 | 0 | 1 | 1 | 0 | 0 | 0 | 0 | 0 | 0 | 0 |
| 2-Decen-1-ol | 0 | 1 | 0 | 1 | 1 | 0 | 3 | 1 | 0 | 0.08 | 0.08 | 0 | 0 | 0.08 | 0 | 0.08 | 0.08 | 0 |
| Naphthalene | 1 | 1 | 1 | 1 | 0 | 0 | 3 | 1 | 0 | 0.08 | 0.08 | 0 | 0.08 | 0.08 | 0.08 | 0.08 | 0 | 0 |
| Diethyl Phthalate | 1 | 0 | 1 | 1 | 1 | 0 | 3 | 1 | 0 | 0.08 | 0.08 | 0 | 0.08 | 0 | 0.08 | 0.08 | 0.08 | 0 |
| Indice 3I | | | | | | | | | | | | | 2.72 | 0.48 | 2.48 | 3.8 | 4.64 | 5.4 |

**Calcul de la valeur (VL)**

La valeur limite VL a été calculée à l'aide de la formule indiquée dans l'étape d-3/ ci-dessus, à savoir :

$$VL = \frac{\text{Nombre de "COV non pondérés"} + P \times \text{Nombre de "COV pondérés"}}{\text{Coefficient } Z}$$

Il ressort des tableaux 3 et 4 la présence de 13 « COV non pondérés » (car présents uniquement en cas d'infestation) et 23 « COV pondérés ».

Dans ce cas, la valeur VL est égale à 3,71 = [13 + (0,08x23)]/4.

Les valeurs de l'indice (3I), précédemment indiquées dans les tableaux 3 et 4 ci-dessus sont résumées dans le tableau 5 ci-dessous.

Les ambiances et environnements non infestés correspondent respectivement aux 5 premières lignes de la case « Ambiances laboratoire » et aux 3 premières lignes de la case « Environnements in situ ».

## Tableau 5 : valeurs des indices 3I

| Ambiances et environnements | | | Valeur de l'indice 3I |
|---|---|---|---|
| Ambiances laboratoire | Sans infestation | Laine (référence 1) | 2.32 |
| | | Laine (référence 2) | 0.64 |
| | | Laine anciennement infestée 1 | 0.24 |
| | | Laine anciennement infestée 2 | 2.96 |
| | | Peau de lapin (référence) | 1.28 |
| | Avec infestation | **Laine infestée 1** | **4.24** |
| | | **Laineinfestée 2** | **7.88** |
| | | **Laine infestée 3** | **4.80** |
| | | **Laine infestée 4** | **7.20** |
| | | **Peau de lapin infestée** | **9.28** |
| Environnements in situ | Sans infestation | Voiture (extérieur) | 2.72 |
| | | Logement 1 | 0.48 |
| | | Logement 2 | 2.48 |
| | Avec infestation | **Voiture (intérieur)** | **3.80** |
| | | **Palais 1** | **4.64** |
| | | **Palais 2** | **5.40** |

**Conclusion** :

L'ensemble des ambiances et environnements infestés présente, comme attendu, un indice (3I) strictement supérieur à la Valeur Limite VL (dans ce cas 3,71). *A contrario*, l'ensemble des ambiances et environnements non infestés présentent un indice inférieur ou égal à cette même valeur limite.

De plus, les deux ambiances anciennement infestées présentent un indice inférieur à 3,71, traduisant l'absence de COV résiduels une fois l'infestation passée et contrôlée.

L'indice (3I) traduit donc bien l'activité de la mite des vêtements, à savoir *Tineola Bisselliella.*

La méthode de l'invention de construction d'indice (3I) et de valeur VL permet donc avantageusement la détection d'insectes au stade larvaire.

## Revendications

1. Procédé de détection de la présence d'insectes dans un environnement intérieur comprenant :

   a/ le prélèvement d'un échantillon de composés organiques volatils (COV) dans l'environnement intérieur ;
   b/ la séparation des COV prélevés ;
   c/ la détection de la présence ou de l'absence de COV « cibles » prédéterminés, ces COV « cibles » prédéterminés appartenant à au moins l'une des trois catégories de COV suivantes :

   - les COV qui sont émis indépendamment du support sur lequel les insectes se développent et qui ne sont émis que lors d'une infestation par des insectes, dénommés « COV de la catégorie 1 » ;
   - les COV qui sont émis indépendamment du support sur lequel les insectes se développent mais qui peuvent également avoir d'autres origines biologiques que les insectes, dénommés « COV de la catégorie 2 » ;
   - les COV qui sont émis en fonction du support sur lequel les insectes se développent et qui ne sont émis que lors d'une infestation par des insectes, dénommés « COV de la catégorie 3 » ;

   d/ le calcul respectif d'une valeur « Indice Infestation Insecte » dénommée « 3I » (ou « indice 3I ») et d'une valeur « Valeur limite » dénommée « VL », qui sont dépendantes chacune de la présence ou de l'absence des COV cibles prédéterminés,
   e/ la comparaison des valeurs « 3I » et « VL », si la valeur « 3I » est strictement supérieure à la valeur « VL » alors l'environnement est infesté, et si la valeur « 3I » est inférieure ou égale à la valeur « VL » alors l'environnement n'est pas infesté.

2. Procédé de détection de la présence d'insectes selon la revendication 1, **caractérisé en ce que** l'étape **d**/ est réalisée en trois sous-étapes :

    **d-1**/ détermination d'une valeur d'incrémentation « i », la valeur d'incrémentation étant obtenue par attribution à chacun des COV cibles pré-déterminés d'un chiffre de -1, 0 ou 1 en fonction de sa présence ou de son absence, l'attribution des chiffres se faisant de la façon suivante :

        - la présence de COV de la catégorie (1) est **caractérisée par** le chiffre 1 et son absence par -1 ;
        - la présence de COV de la catégorie (2) est **caractérisée par** le chiffre 0 et son absence par -1 ;
        - la présence de COV de la catégorie (3) est **caractérisée par** le chiffre 1 et son absence par 0 ;

    **d-2**/ détermination d'une valeur de pondération « P » (encore dénommée « facteur de pondération ») correspondant à la formule suivante :

$$P = \frac{\text{Coefficient 1}}{\text{Nombre de "COV pondérés"}}$$

    dans laquelle :

        - les « COV pondérés » correspondent aux COV retrouvés sur des supports sans infestation active mais émis en quantité relative plus importante en présence des insectes, à savoir en une quantité au moins deux fois supérieure,
        - le coefficient 1 est un nombre réel empirique fixé par itération avec un panel d'environnements dont l'infestation est connue ;

    **d-3**/ détermination de la valeur 3I et VL,

        - la valeur (3I) correspondant à la formule suivante :

$$(3I) = \sum_{j=n}^{1} i_j \times P_j$$

    dans laquelle :

        n représente le nombre de COV cibles,
        j représente un nombre entier,
        i représente la valeur d'incrémentation telle que définie à l'étape d-1/,
        P représente la valeur de pondération telle que définie à l'étape d-2/,
        $i_j$ représente l'incrémentation du $j^{ème}$ COV cible,
        $P_j$ représente la pondération du $j^{ème}$ COV cible,

        - la valeur (VL) correspondant à la formule suivante :

$$VL = \frac{\text{Nombre de "COV non pondérés"} + P \times \text{Nombre de "COV pondérés"}}{Coefficient\ 2}$$

    dans laquelle les « COV non pondérés » correspondent aux COV émis uniquement en présence d'insectes, le coefficient 2 est un nombre réel empirique fixé par itération avec le même panel d'environnement que celui utilisé pour la détermination du « coefficient 1 ».

3. Procédé de détection de la présence d'insectes selon la revendication 1 ou la revendication 2, **caractérisé en ce que** les COV cibles prédéterminés sont choisis dans le groupe comprenant le 2,2'-bi-1,3-dioxolane ; l'acétate de n-butyle ; le 1,3,5,7-cyclooctatetraene ; l'alpha-pinène ; le 1-propanol ; l'acide méthane carbothiolique ; le 3,3-dimethyl-1-hexene ; le 2-butanone ; l'acétate d'éthyle ; le 4-hydroxy-2-butanone ; le 1-butanol ; le 2,5-diméthyl-furane ; le 2-éthényl-2-buténal ; le butyle formate ; la pyrazine ; le pyrrole ; le 1-pentanol ; le 1-(3,4-diméthylthiéno[2,3-b]thiophèn-2-yl)-éthanoneoxime ; le 1,4-octadiène ; le 1,3-octadiène ; le 2-méthyl-pyrimidine ; le 1-hexanol ; le 2-heptanone ; le 2,5-diméthyl-pyrazine ; le camphène ; le 2-octanone ; le 1-méthyl-4-(1-méthyléthényl)-cyclohéxène ; le 2-méthyl-décane ; l'acétophénone ; le 2-nonèn-1-ol ; le 1,10-dichlorodécane ; le dodécanal ; le 2-pentyl-thiophène ; le 2-decène-1-ol ; le naphtalène ; le diéthyl phthalate, et leurs mélanges.

4. Procédé de détection de la présence d'insectes selon l'une quelconque des revendications 1 à 3, **caractérisé en ce que** :

> - les COV cibles prédéterminés de la catégorie 1 sont choisis dans le groupe comprenant le 2,2'-bi-1,3-dioxolane et l'acétate de n-butyle ;
> - les COV cibles prédéterminés de la catégorie 2 sont choisis dans le groupe comprenant le 1,3,5,7-cyclooctatetraene et l'alpha-pinène ;
> - les COV cibles prédéterminés de la catégorie 3 sont choisis dans le groupe comprenant le 1-propanol ; l'acide méthane carbothiolique ; le 3,3-dimethyl-1-hexene ; le 2-butanone ; l'acétate d'éthyle ; le 4-hydroxy-2-butanone ; le 1-butanol ; le 2,5-diméthyl-furane ; le 2-éthényl-2-buténal ; le butyle formate ; la pyrazine ; le pyrrole ; le 1-pentanol ; le 1-(3,4-diméthylthiéno[2,3-b]thiophèn-2-yl)-éthanoneoxime ; le 1,4-octadiène ; le 1,3-octadiène ; le 2-méthyl-pyrimidine ; le 1-hexanol ; le 2-heptanone ; le 2,5-diméthyl-pyrazine ; le camphène ; le 2-octanone ; le 1-méthyl-4-(1-méthyléthényl)-cyclohéxène ; le 2-méthyl-décane ; l'acétophénone ; le 2-nonèn-1-ol ; le 1,10-dichlorodécane ; le dodécanal ; le 2-pentyl-thiophène ; le 2-decène-1-ol ; le naphtalène et le diéthyl phthalate.

5. Procédé de détection de la présence d'insectes selon l'une quelconque des revendications 1 à 4, **caractérisé en ce que** les insectes sont à l'état de larves.

6. Procédé de détection de la présence d'insectes selon l'une quelconque des revendications 2 à 5, **caractérisé en ce que** :

> - l'insecte est *Tineola bisselliella*,
> - le coefficient 1 présente une valeur allant de 1 à 3, et est de préférence égal à 2,
> - le coefficient 2 présente une valeur allant de 3 à 5, et est de préférence égal à 4.

**Patentansprüche**

1. Verfahren zum Nachweis der Anwesenheit von Insekten in einer Innenraumumgebung, umfassend:

> a/ Entnahme einer Probe flüchtiger organischer Verbindungen (VOCs) aus der Innenraumumgebung;
> b/ Abtrennung der entnommenen VOCs;
> c/ Erfassen der Anwesenheit oder Abwesenheit von vorbestimmten "Ziel"-VOCs, wobei die vorbestimmten "Ziel"-VOCs zu mindestens einer der folgenden drei Kategorien von VOCs gehören:
>
> > - VOCs, die unabhängig von dem Träger, auf dem sich die Insekten entwickeln, emittiert werden und die nur bei Insektenbefall emittiert werden, so genannte "VOCs der Kategorie 1";
> > - VOCs, die unabhängig von dem Träger, auf dem sich die Insekten entwickeln, emittiert werden, die aber auch andere biologische Ursprünge haben können als Insekten, so genannte "VOCs der Kategorie 2";
> > - VOCs, die in Abhängigkeit von dem Träger, auf dem sich die Insekten entwickeln, emittiert werden und die nur bei Insektenbefall emittiert werden, so genannte "VOCs der Kategorie 3";
>
> d/ jeweilige Berechnung eines "Insektenbefallsindex"-Wertes mit der Bezeichnung "3I" (oder "Index 3I") und eines "Grenzwert"-Wertes mit der Bezeichnung "VL", die jeweils von der Anwesenheit oder Abwesenheit der vorher festgelegten Ziel-VOCs abhängig sind,
> e/ Vergleich der Werte "3I" und "VL", wenn der Wert "3I" strikt über dem Wert "VL" liegt, dann ist die Umgebung befallen, und wenn der Wert "3I" kleiner oder gleich dem Wert "VL" ist, dann ist die Umgebung nicht befallen.

2. Verfahren zum Nachweis der Anwesenheit von Insekten nach Anspruch 1, **dadurch gekennzeichnet, dass** Schritt d/ in drei Unterschritten durchgeführt wird:

> d-1/ Bestimmung eines Inkrementierungswerts "i", wobei der Inkrementierungswert dadurch erhalten wird, dass jeder der vorbestimmten Ziel-VOCs eine Zahl von -1, 0 oder 1 zugeordnet wird, je nachdem, ob sie vorhanden sind oder nicht, wobei die Zuordnung der Zahlen folgendermaßen erfolgt:

- die Anwesenheit von VOCs der Kategorie (1) wird mit der Zahl 1 und ihre Abwesenheit mit -1 gekennzeichnet;
- die Anwesenheit von VOCs der Kategorie (2) wird mit der Zahl 0 und ihre Abwesenheit mit-1 gekennzeichnet;
- die Anwesenheit von VOCs der Kategorie (3) wird mit der Zahl 1 und ihre Abwesenheit mit 0 gekennzeichnet;

d-2/ Bestimmung eines Gewichtungswertes "P" (auch als "Gewichtungsfaktor" bezeichnet), der der folgenden Formel entspricht:

$$P = \frac{\text{Koeffizient 1}}{\text{Anzahl der "gewichteten VOCs "}}$$

worin:

- die "gewichteten VOCs" den VOCs entsprechen, die auf Trägern ohne aktiven Befall gefunden werden, die aber bei Anwesenheit von Insekten in einer größeren relativen Menge emittiert werden, d.h. in einer mindestens doppelt so großen Menge,
- der Koeffizient 1 eine empirisch ermittelte reelle Zahl ist, die durch Iteration mit einem Panel von Umgebungen mit bekanntem Befall festgelegt wird;

d-3/ Bestimmung des Wertes 3I und VL,

- wobei der Wert (3I) der folgenden Formel entspricht:

$$(3I) = \sum_{j=n}^{1} i_j \times P_j$$

worin:

n die Anzahl der Ziel-VOCs darstellt,
j eine ganze Zahl darstellt,
i den Inkrementierungswert wie in Schritt d-1/ definiert darstellt,
P den Gewichtungswert wie in Schritt d-2/ definiert darstellt,
$i_j$ die Inkrementierung der j-ten Ziel-VOC darstellt,
$P_j$ den Gewichtungswert der j-ten Ziel-VOC darstellt,

- der Wert (VL) der der folgenden Formel entspricht:

$$VL = \frac{\text{Anzahl der "nicht gewichteten VOCs" + P x Anzahl der "gewichteten VOCs"}}{\text{Koeffizient 2}}$$

worin die "nicht gewichteten VOC" den VOCs entsprechen, die nur in Gegenwart von Insekten emittiert werden, Koeffizient 2 eine empirische reelle Zahl ist, die durch Iteration mit demselben Umgebungspanel wie dem zur Bestimmung von "Koeffizient 1" verwendeten Panel festgelegt wird.

3. Verfahren zum Nachweis der Anwesenheit von Insekten nach Anspruch 1 oder 2, **dadurch gekennzeichnet, dass** die vorbestimmten Ziel-VOCs ausgewählt sind aus der Gruppe umfassend: 2,2'-Bis-1,3-dioxolan; n-Butylacetat;1,3,5,7-Cyclooctatetraen; alpha-Pinen; 1-Propanol; Methan-Carbothiolsäure; 3,3-Dimethyl-1-hexen; 2-Butanon; Ethylacetat; 4-Hydroxy-2-butanon; 1-Butanol; 2,5-Dimethylfuran; 2-Ethenyl-2-butenal; Butylformiat; Pyrazin; Pyrrol; 1-Pentanol; 1-(3,4-Dimethylthien[2,3-b]thiophen-2-yl)-ethanonoxim; 1,4-Octadien; 1,3-Octadien; 2-Methyl-pyrimidin; 1-Hexanol; 2-Heptanon; 2,5-Dimethyl-pyrazin; Camphen; 2-Octanon; 1-Methyl-4-(1-methylethylenyl)cyclohexen; 2-Methyldecan; Acetophenon; 2-Nonen-1-ol; 1,10-Dichlordecan; Dodecanal; 2-Pentyl-thiophen; 2-Decen-1-ol; Naphthalin; Diethylphthalat und Mischungen davon.

4. Verfahren zum Nachweis der Anwesenheit von Insekten nach einem der Ansprüche 1 bis 3, **dadurch gekennzeichnet, dass**:

   - die vorbestimmten Ziel-VOCs der Kategorie 1 aus der Gruppe ausgewählt sind, die 2,2'-Bis-1,3-dioxolan und n-Butylacetat umfasst;
   - die vorbestimmten Ziel-VOCs der Kategorie 2 aus der Gruppe ausgewählt sind, die 1,3,5,7-Cyclooctatetraen und alpha-Pinen umfasst;
   - die vorbestimmten Ziel-VOCs der Kategorie 3 aus der Gruppe ausgewählt sind, die 1-Propanol; Methan-Carbothiolsäure; 3,3-Dimethyl-1-hexen; 2-Butanon; Ethylacetat; 4-Hydroxy-2-butanon; 1-Butanol; 2,5-Dimethylfuran; 2-Ethenyl-2-butenal; Butylformiat; Pyrazin; Pyrrol; 1-Pentanol; 1-(3,4-Dimethylthien[2,3-b]thiophen-2-yl)-ethanonoxim; 1,4-Octadien; 1,3-Octadien; 2-Methylpyrimidin; 1-Hexanol; 2-Heptanon; 2,5-Dimethylpyrazin; Camphen; 2-Octanon; 1-Methyl-4-(1-methylethenyl)cyclohexen; 2-Methyldecan; Acetophenon; 2-Nonen-1-ol; 1,10-Dichlordecan; Dodecanal; 2-Pentyl-thiophen; 2-Decen-1-ol; Naphthalin und Diethylphthalat umfasst.

5. Verfahren zum Nachweis der Anwesenheit von Insekten nach einem der Ansprüche 1 bis 4, **dadurch gekennzeichnet, dass** sich die Insekten im Larvenstadium befinden.

6. Verfahren zum Nachweis der Anwesenheit von Insekten nach einem der Ansprüche 2 bis 5, **dadurch gekennzeichnet, dass**:

   - es sich bei dem Insekt um *Tineola bisselliella* handelt,
   - der Koeffizient 1 einen Wert von 1 bis 3 aufweist und vorzugsweise gleich 2 ist,
   - der Koeffizient 2 einen Wert von 3 bis 5 aufweist und vorzugsweise gleich 4 ist.

**Claims**

1. A method of detecting the presence of insects in an indoor environment comprising:

   a/ taking a sample of volatile organic compounds (VOCs) in the indoor environment;
   b/ separating the VOCs sampled;
   c/ detecting the presence or absence of predetermined "target" VOCs, these predetermined "target" VOCs belonging to at least one of the following three categories of VOCs:

      - VOCs that are emitted independently of the substrate on which the insects grow, and that are only emitted during an insect infestation, called "category 1 VOCs";
      - VOCs that are emitted independently of the substrate on which the insects grow but that may also have biological origins other than insects, called "category 2 VOCs";
      - VOCs that are emitted as a function of the substrate on which the insects grow, and that are only emitted during an insect infestation, called "category 3 VOCs";

   d/ calculating respectively a value "Insect Infestation Index" called "31" (or "3I index") and a value "Limit Value" called "VL", each of which depends on the presence or absence of the predetermined target VOCs,
   e/ comparing the values "31" and "VL"; if the value "31" is strictly greater than the value "VL" then the environment is infested, and if the value "31" is less than or equal to the value "VL", the environment is not infested.

2. The method of detecting the presence of insects as claimed in claim 1, **characterized in that** step d/ is carried out in three substeps:

   d-1/ determining an incrementing value "i", the incrementing value being obtained by assigning, to each of the predetermined target VOCs, a number -1, 0 or 1 depending on its presence or its absence, the numbers being assigned as follows:

      - presence of a VOC of category (1) is **characterized by** the number 1 and its absence by -1;
      - presence of a VOC of category (2) is **characterized by** the number 0 and its absence by -1;

- presence of a VOC of category (3) is **characterized by** the number 1 and its absence by 0;

**d-2/** determining a weighting value "P" (also called "weighting factor") corresponding to the following formula:

$$P = \frac{\text{Coefficient 1}}{\text{Number of "weighted VOCs"}}$$

in which:

- the "weighted VOCs" correspond to the VOCs found on substrates without active infestation but emitted in a larger relative amount in the presence of the insects, namely in an amount at least two times higher,
- coefficient 1 is an empirical real number established by iteration with a panel of environments whose infestation is known;

**d-3/** determining the value 31 and VL,

- the value (31) corresponding to the following formula:

$$(3I) = \sum_{j=n}^{1} i_j \times P_j$$

in which:

n represents the number of target VOCs,
j represents an integer,
i represents the incrementing value as defined in step d-1/,
P represents the weighting value as defined in step d-2/,
$i_j$ represents the incrementation of the j-th target VOC,
$P_j$ represents the weighting of the j-th target VOC,

- the value (VL) corresponding to the following formula:

$$VL = \frac{\text{Number of "unweighted VOCs" + P x Number of "weighted VOCs"}}{\textit{Coefficient 2}}$$

in which the "unweighted VOCs" correspond to the VOCs only emitted in the presence of insects, coefficient 2 is an empirical real number established by iteration with the same environment panel as that used for determining "coefficient 1".

3. The method of detecting the presence of insects as claimed in claim 1 or claim 2, **characterized in that** the predetermined target VOCs are selected from the group comprising 2,2'-bi-1,3-dioxolane; n-butyl acetate; 1,3,5,7-cyclooctatetraene; alpha-pinene; 1-propanol; methanecarbothiolic acid; 3,3-dimethyl-1-hexene; 2-butanone; ethyl acetate; 4-hydroxy-2-butanone; 1-butanol; 2,5-dimethyl-furan; 2-ethenyl-2-butenal; butyl formate; pyrazine; pyrrole; 1-pentanol; 1-(3,4-dimethylthieno[2,3-b]thiophen-2-yl)-ethanone-oxime; 1,4-octadiene; 1,3-octadiene; 2-methyl-pyrimidine; 1-hexanol; 2-heptanone; 2,5-dimethyl-pyrazine; camphene; 2-octanone; 1-methyl-4-(1-methylethenyl)-cyclohexene; 2-methyldecane; acetophenone; 2-nonen-1-ol; 1,10-dichlorodecane; dodecanal; 2-pentyl-thiophene; 2-decen-1-ol; naphthalene; diethyl phthalate, and mixtures thereof.

4. The method of detecting the presence of insects as claimed in any one of claims 1 to 3, **characterized in that**:

- the predetermined target category 1 VOCs are selected from the group comprising 2,2'-bi-1,3-dioxolane and n-butyl acetate;
- the predetermined target category 2 VOCs are selected from the group comprising 1,3,5,7-cyclooctatetraene and alpha-pinene;
- the predetermined target category 3 VOCs are selected from the group comprising 1-propanol; methanecarbothiolic acid; 3,3-dimethyl-1-hexene; 2-butanone; ethyl acetate; 4-hydroxy-2-butanone; 1-butanol; 2,5-dimethyl-furan; 2-ethenyl-2-butenal; butyl formate; pyrazine; pyrrole; 1-pentanol; 1-(3,4-dimethylthieno[2,3-b]thiophen-2-yl)-ethanone-oxime; 1,4-octadiene; 1,3-octadiene; 2-methyl-pyrimidine; 1-hexanol; 2-heptanone; 2,5-dimethyl-pyrazine; camphene; 2-octanone; 1-methyl-4-(1-methylethenyl)-cyclohexene; 2-methyldecane; acetophenone; 2-nonen-1-ol; 1,10-dichlorodecane; dodecanal; 2-pentyl-thiophene; 2-decen-1-ol; naphthalene and diethyl phthalate.

**5.** The method of detecting the presence of insects as claimed in any one of claims 1 to 4, **characterized in that** the insects are in the larval state.

**6.** The method of detecting the presence of insects as claimed in any one of claims 2 to 5, **characterized in that**:

- the insect is *Tineola bisselliella*,
- coefficient 1 has a value from 1 to 3, and is preferably equal to 2,
- coefficient 2 has a value from 3 to 5, and is preferably equal to 4.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2913501

**Littérature non-brevet citée dans la description**

- **SAI XU et al.** Récognition of the Duration and Prediction of Insect Prevalence of Stored Rough Rice Infested by the Red Flour Beetle (Tribolium castaneum Herbst) Using an Electronic Nose. *SENSORS,* 14 Avril 2017, vol. 17 (4), 688